# EUROPEAN PATENT SPECIFICATION

(11) **EP 3 099 659 B1**
(45) Date of publication and mention of the grant of the patent: **14.03.2018**
(21) Application number: 15701344.2
(22) Date of filing: 26.01.2015
(51) Int. Cl.: C07C 41/09, C07C 41/42, C07C 43/04, B01D 3/00, B01D 3/14, B01D 3/16

(54) **PROCESS AND INSTALLATION FOR THE PRODUCTION OF DIALKYL ETHER**
VERFAHREN UND ANLAGE ZUR HERSTELLUNG VON DIALKYLETHER
PROCÉDÉ ET INSTALLATION POUR LA PRODUCTION DE L'ÉTHER DE DIALKYLE

(30) Priority: 28.01.2014 EP 14152850
(43) Date of publication of application: 07.12.2016
(73) Proprietor: Akzo Nobel Chemicals International B.V., 6824 BM Arnhem (NL)
(72) Inventor: KISS, Anton, Alexandru, NL-6835 CM Arnhem (NL); BILDEA, Costin, Sorin, R-010798 Bucuresti (RO); PATRUT, Catalin, R-230025 Slatina (RO)
(74) Representative: Akzo Nobel IP Department
(86) International application number: PCT/EP2015/051425
(87) International publication number: WO 2015/113914

(56) References cited:
- US-A1- 2009 069 607
- VLADIMIR N MALETA ET AL: "Understanding process intensification in cyclic distillation systems", CHEMICAL ENGINEERING AND PROCESSING, ELSEVIER SEQUOIA, LAUSANNE, CH, vol. 50, no. 7, 4 April 2011 (2011-04-04), pages 655-664, XP028240261, ISSN: 0255-2701, DOI: 10.1016/J.CEP.2011.04.002 [retrieved on 2011-04-16] cited in the application

## Description

The present invention pertains to a process and an installation for the production of a dialkyl ether by the partial dehydration of C1-C3 alcohols. A specific example is the conversion of methanol in the production of dimethyl ether.

Conventionally dimethyl ether is produced by the catalytic dehydration of gas-phase methanol in a pipe reactor. The product is cooled and distilled to yield a dimethyl ether of sufficient purity.

It has been proposed to intensify processes involving conversion and distillation steps, for instance by so-called reactive or catalytic distillation. Zhigang Lei c.s., Synthesis of dimethyl ether (DME) by catalytic distillation, Chemical Engineering Science, 66 (2011), pp. 3195 - 3203, discloses a process that makes use of a reactive distillation column for the conversion of methanol to dimethyl ether. Conversion and distillation take place in the same column. The produced dimethyl ether is distilled and a mixture of water and methanol is fed into a next distillation column. The disclosed process requires at least 30 stages or an upstream fixed-bed reactor.

Maleta c.s., Understanding process intensification in cyclic distillation systems, Chemical Engineering and Processing, 50 (2011), pp. 655 - 664, discloses so-called cyclic distillation. A cyclic distillation column has an operating cycle including a vapour flow period - when vapour flows upwards through the column and liquid remains stationary on each plate - and a liquid flow period when the vapour flow is stopped, reflux and feed liquid are supplied, and the liquid flows down from each tray to the one below, by gravity. Catalytic cyclic distillation is also mentioned. Due to the pressure drop at each stage, the number of stages that can be used is typically relatively low, generally for example 15.

US 2009/069607 discloses a process for the production of dialkyl ether comprising feeding a stream comprising an alkyl alcohol to a distillation column reactor system, concurrently in the distillation column reactor system (i) contacting the alkyl alcohol with a catalytic distillation structure in a distillation reaction zone thereby catalytically reacting at least a portion of the alkyl alcohol to form the corresponding dialkyl ether and water and (ii) fractionating the resulting dialkyl ether from the water; operating the distillation column reactor system to obtain substantially complete conversion of the alkyl alcohol to form the corresponding dialkyl ether and water; recovering the dialkyl ether from the distillation column reactor as an overheads fraction; recovering the water from the distillation column reactor as a bottoms fraction.

Although reactive distillation results in an improved and intensified production process, there is still a continuous need to achieve yields with higher purities. Hence, the object of the present invention is to provide a process resulting in yields of higher purity.

The object of the invention is achieved with a process for the production of dialkyl ether wherein an alcohol is fed into a cyclic distillation column comprising a reboiler at its bottom, a condenser at its top, a feed inlet at a level between the reboiler and the condenser, and a stack of stages in the column's interior, including stages carrying a bed of catalytic material. The stages comprise sluices which per stage are simultaneously moved between a vapour flow position, allowing vapour to flow upwards through the catalytic beds during a vapour flow period, and a liquid flow position, allowing liquid to flow from an upper stage to a lower stage during a liquid flow period. The alcohol according to the present invention is or comprises a C1-C3 alcohol (i.e. methanol, ethanol, 1-propanol or 2-propanol). Accordingly, the dialkyl ether produced via the process according to the present invention is dimethyl ether, diethyl ether, dipropyl ether or diisopropyl ether. It is noted that the phrase "the alcohol comprises a C1-C3 alcohol" is meant to denote mixtures comprising at least 70% by weight of said alcohol, preferably at least 80% by weight of said alcohol, more preferably at least 90% by weight of said alcohol, more preferably still at least 95% by weight of said alcohol, and most preferably at least 97% by weight of said alcohol. The phrase "the alcohol is a C1-C3 alcohol" is meant to denote C1-C3 alcohols with a purity of at least 98%.

It has been found that such periodic or cyclic operation results in very high conversion rates and yields of very high purity, even with a relatively low number of stages, e.g., less than 25 stages.

The stages may for example comprise a tray with an upper tray floor and a lower tray floor with aligned openings, while the sluices comprise a plunger selectively closing off the opening in the upper tray floor or the associated aligned opening in the lower tray floor. This prevents mixing of liquids of different stages. It has been found that this substantially increases the performance of the distillation unit.

A specific example of a distillation column particularly suitable for periodic or cyclic operation is disclosed in US 2010/0221156.

The catalytic cyclic distillation column may for example comprise a rectifying section with rectifying stages at its top, a stripping section with stripping stages at the bottom, and reactive stages between the stripping and rectifying sections. The reactive stages may comprise catalytic material. In the rectifying stages, alcohol is separated from the dialkyl ether vapour, while in the stripping stages, water is separated from the alcohol.

The alcohol feed enters the column at the level of the reactive stages. If the alcohol is methanol, it will typically be fed to the column as a saturated liquid phase. The alcohol passes the catalytic material of the reactive stages and is dehydrated to form a mixture of dialkyl ether and water. The liquid mixture of water with unreacted alcohol flows down to the stripping section, where the alcohol is stripped from the water and returned with an upward vapour flow. The vapour mixture of dialkyl ether with unreacted alcohol flows upwards into the rectifying section, where the temperature conditions are such that liquid alcohol is partly separated from the dialkyl ether vapour. If the condenser is a partial condenser, vapour leaving the rectifying section is partially condensed. The condensed liquid is then returned as a reflux to the column while uncondensed vapour is discharged, e.g. with a dialkyl ether purity exceeding 50% molar. Alternatively, a total condenser can be used, so the vapour leaving the rectifying section is completely condensed. In that case a part of the condensed liquid is returned as a reflux to the column, while the rest of the condensed liquid is discharged. Optionally, the discharged dialkyl ether can be fed into a next distillation column, e.g., a cyclic or a non-cyclic distillation column, for further purification and separation of alcohol.

The bottom of the cyclic distillation column is provided with a reboiler. The separated mixture of water and alcohol is reboiled. Alcohol vapour is returned to the column while purified liquid water is discharged, e.g., with a purity exceeding 50% molar. Optionally, the discharged water can be fed into a next distillation column, e.g., a cyclic or a non-cyclic distillation column, for further purification of water.

Inside the column, the reflux alcohol flows downwards and provides cooling and partial condensation of the upflowing vapours, thereby increasing the efficacy of the distillation tower. A stronger reflux flow improves the column's separation of the higher boiling alcohol and water from the lower boiling dialkyl ether. A balance of heating by a reboiler at the bottom of a column and cooling by condensed reflux at the top of the column maintains a temperature gradient along the height of the column to provide good conditions for dehydration of the alcohol component.

The reflux amount at the condenser and the vapour flow rate at the reboiler can be used to improve and control the separation performance of the distillation column. The reflux amount and the vapour flow rate can be chosen such that a high conversion is combined with high purities of the distillate and the water. The vapour flow rate is defined as the flow of vapour generated by the reboiler during the vapour flow period. For instance, for a feed amount of 0.15 kmol per cycle, the reflux can be at least 3.5 kmol per cycle, or at least 4 kmol mol per cycle or at least 4.3 kmol per cycle and the vapour flow rate can for instance be at least 1 kmol/min, or at least 1.5 kmol/min or at least 2 kmol/min for a duration of 0.5 to 2 min.

To obtain a product of the desired purity the reflux amount and the vapour flow rate should be balanced with other process parameters including pressure, temperature, and the number of reactive, stripping, and rectifying stages required.

The distillation column can for example comprise:
- at least 2, e.g. at most 5, for instance 3 or 4 rectifying stages;
- at least 5, e.g. at most 9, for instance 7 or 8 reactive stages;
- at least 4, e.g. at most 9, for instance 6 or 7 stripping stages.

A particular example would for instance have 3 rectifying stages, 8 reactive stages with about 10 kg per stage of catalyst, such as Amberlyst® 35, and 6 stripping stages. Such a column could for example be fed with about 0.15 kmol/cycle of methanol, with a cycle duration of 0.2 min, and be operated at about 10 bar and:
- a temperature of 395 K - 405 K in the top of the column and of 450 - 455 K in the bottom of the column, if the distillate contains a high concentration of dimethyl ether and the bottom product is essentially pure water (more than 99.9% molar purity);
- or a temperature of 315 K - 320 K in the top of the column and of 435 - 445 K in the bottom of the column, if the distillate is essentially pure dimethyl ether (more than 99.9% molar purity) and the bottom product contains a mixture of water and methanol;
- a temperature of 315 K - 320 K in the top of the column and of 450 K - 455 K in the bottom of the column, if the distillate is essentially pure dimethyl ether (more than 99.9% molar purity) and the bottom product is essentially pure water (more than 99.9% molar purity).

The reflux amount and the vapour flow rate can be controlled, for example by means of automated control, to maintain a low temperature in the top of the column (for example, 395 K - 405 K on the top tray of the rectifying section) and a higher temperature in the bottom of the column (for example, 450 K - 455 K on the last tray of the stripping section). It has been found that in such case, with a reflux amount of about 3.8 - 4.7 kmol per cycle, a vapour flow rate of 1.5 kmol/min and a vapour-flow period of 1 min, essentially pure water (more than 99.9 % molar purity) can be discharged from the reboiler, which does not need further purification. The alkyl ether content of the distillate discharged from the condenser can be higher than 80% molar. If so desired, the distillate can be further purified in a next distillation column.

Another particular example would for instance have 3 rectifying stages, 8 reactive stages reactive stages with 10 kg per stage of catalyst Amberlyst® 35, and 6 stripping stages. The column would be fed with 0.15 kmol/cycle of methanol and would be operated around 10 bar. The reflux amount and the vapour flow rate would be controlled, for example by means of automated control, to maintain a low temperature in the top of the column (for example, 315 K - 320 K on the top tray of the rectifying section) and a high temperature in the bottom of the column (for example, 435 K - 445 K on the last tray of the stripping section). It has been found that in such case, with a reflux amount of about 3.8 - 4.7 kmol per cycle, a vapour flow rate of 1.5 kmol/min and a vapour-flow period of 1 min, essentially pure alkyl ether (more than 99.9% molar purity) can be discharged from the condenser, which does not need further purification. The water content of the product discharged from the reboiler can be higher than 50% molar. If so desired, the bottom product may be further purified in a next distillation column.

This particular cyclic distillation column can also be configured for full conversion of the methanol feed by setting the reflux amount and the vapour flow rate to such values that equal amounts of dialkyl ether and water are obtained during each cycle with a temperature of 315 - 320 K on the top tray of the rectifying section and a temperature of 450 - 455 K on the bottom tray from the stripping section. The reflux amount could for example be 4.75 kmol/cycle, while the vapour flow rate would be 1.5 kmol/min.

Suitable catalytic materials can be homogeneous or heterogeneous catalysts, for example solid acid catalysts, such as γ-alumina (optionally modified), H-ZSM-5, zeolites or ion exchange resins, such as macroporous sulfonic acid ion exchange resin, e.g., available under the names of Amberlyst® or Nafion®. The catalysts can for example be granulates or particles or they can be used in so-called Texas Teabags or packings, such as Katapak-S® solid packing of Sulzer Chemtech.

The process of the invention is particularly suitable for the production of dimethyl ether by dehydration of methanol.

It has been found that the process is governed by the following groups of process variables: *V·t*ᵥₐₚ/*F* and *t*ᵥₐₚ*m_{cat}/*F*, where V, tvap, F and meat are the vapour flow rate (in kmol/min), duration of the vapour flow period (in min/cycle), feed amount (kmol/cycle) and amount of catalyst on each tray (kg), respectively. When the catalysts described in S. Hossenininejad, A. Afacan, R.E. Hayes, "Catalytic and kinetic study of methanol dehydration to dimethyl ether", Chem. Eng. Res. Des. 90 (2012) 825-833 is used, suitable values for these amounts are *V·t*ᵥₐₚ/*F* = *10* kmol/kmol and *t*ᵥₐₚ*m_{cat}/*F* = 66.6 min kg/kmol. The particular values of the individual variables *V*, *t*ᵥₐₚ, *F* and m_{cat} can be chosen such that hydrodynamic constraints are fulfilled. For example, *V* = 7.5 kmol/min, *t*ᵥₐₚ = 0.2 min, *F*= 0.15 kmol/cycle, m_{cat} = 50 kg.

The present application furthermore pertains to an installation for the production of dialkyl ether wherein a C1-C3 alcohol is fed into a distillation column comprising a reboiler (5) at its bottom, a condenser (4) at its top, a feed inlet (11) at a level between the reboiler and the condenser, and a stack of stages (6) in the column's interior, including stages (8) carrying a bed of catalytic material, the stages comprising sluices (19) which are simultaneously moved between a vapour flow position, allowing vapour to flow upwards through the catalytic beds, and a liquid flow position, allowing fluid to flow from an upper stage to a lower stage.

The invention will be further explained with reference to the accompanying drawings.
- Figure 1:: schematically shows an installation for the conversion of methanol to dimethyl ether;
- Figure 2:: shows an alternative installation for the conversion of methanol to dimethyl ether;
- Figure 3:: shows a further alternative installation for the conversion of methanol to dimethyl ether;
- Figure 4A-C:: shows the interior of the reactive distillation column of the installation of Figure 1, Figure 2 or Figure 3 in three different cyclic stadia.

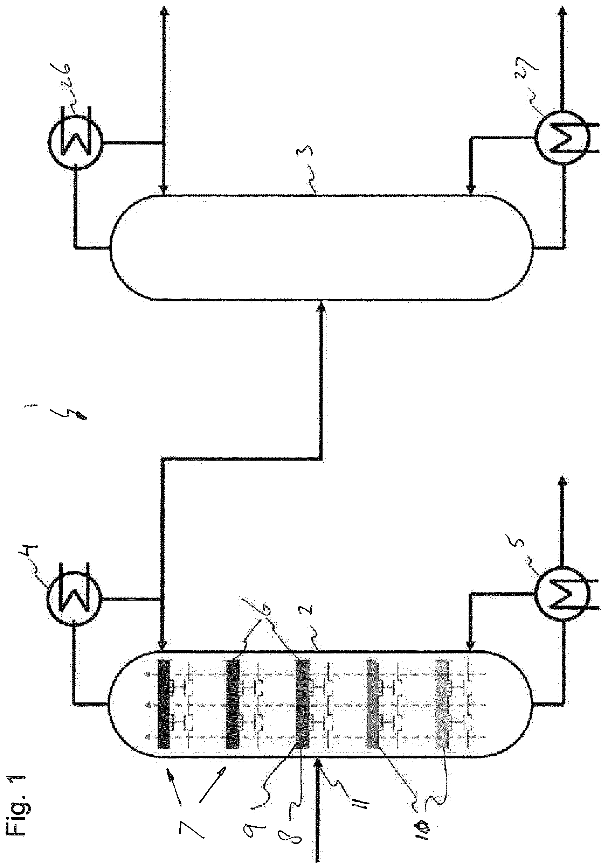

Figure 1 shows an installation 1 comprising a catalytic cyclic distillation column 2 and a second distillation column 3. The catalytic cyclic distillation column 2 comprises a top provided with a condenser 4 and a bottom provided with a reboiler 5. The interior of the catalytic cyclic distillation column 2 comprises a stack of stages 6. At the top the stages 6 include rectifying stages 7 for separating methanol from the dimethyl ether vapour. In the middle part the stages include reactive stages 8 (only one shown in the drawing) comprising catalytic material 9, e.g., 5 - 20 kg, e.g. 8 - 12 kg, e.g., about 10 kg of catalytic material 9 per reactive stage 8. At the bottom the column includes stripping stages 10. While Figure 1 shows only a limited number of stages, the real number of stages will be somewhat larger. In the shown embodiment, the column 2 may for instance have three rectifying stages 7, eight reactive stages 8, and six stripping stages 10.

The column 2 comprises a feed inlet 11 at the level of the reactive stages 8. Methanol in a liquid phase is fed into the column via the feed inlet 11. Methanol passes the catalytic material 9 of the reactive stages 8 and is dehydrated to form a mixture of dimethyl ether and water. The liquid mixture of water with unreacted methanol flows down to the stripping stages 10, where methanol is stripped from the water by vapour rising from the reboiler and returned with an upward vapour flow. The vapour mixture of dimethyl ether with unreacted methanol flows upwards, passing the rectifying stages 7 where the temperature conditions are such that liquid methanol is partly separated from the dimethyl ether vapour. If a partial condenser is used, the distillate will be a vapour distillate. If a total condenser is used, the distillate will be liquid. The vapour or liquid distillate has a low methanol content and is discharged. A reflux of condensed liquid is returned to the rectifying stages 7 in the column 2. In the embodiment of Figure 1 the reflux is methanol or a mixture of dimethyl ether with methanol.

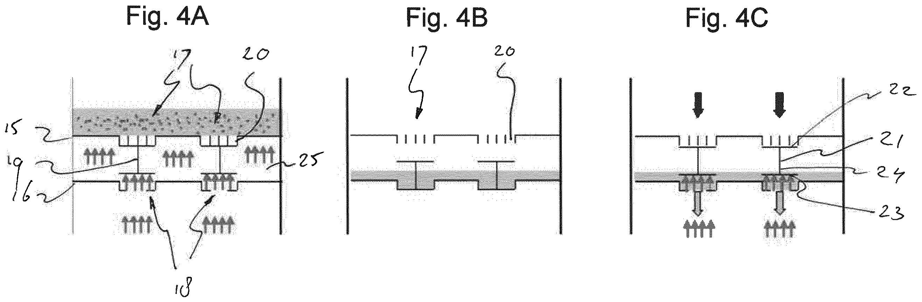

As is shown in more detail in Figures 4A - C, the stages 6 of the cyclic catalytic distillation column 2 are trays comprising an upper tray floor 15 and a lower tray floor 16 with vertically aligned openings 17, 18 selectively closable by sluices 19. The openings 17 in the upper tray floor 15 comprise a downwardly extending collar 20 with openings. The sluices 19 can be moved simultaneously between a vapour flow position (see Figures 4A and 4C), allowing vapour to flow upwards through the stages, and a liquid flow position (see Figure 4B), allowing liquid to flow down from an upper stage 6 to a lower stage 6. The openings in the collar 20 are dimensioned such that, when the sluices are in the vapour flow position, vapour passes through these openings. The pressure exerted by the vapour flow prevents liquid from flowing down through these openings.

The sluices 19 comprise a plunger 21 with a horizontal upper flange 22, a horizontal lower flange 23, and a shaft 24 connecting the two flanges. In Figure 4A the plungers 21 are moved into an upward position, closing off the passage openings 17 in the upper tray floor 15 except for the openings in the collar 20, while the openings 18 in the lower tray floor 16 are open. In that position of the sluices 19 vapour can flow upwards from a lower stage 6 via the space 25 between the upper and the lower tray floor through the permeable collar 20 to a next upper stage. Liquid on the stage 6 remains stagnant on the upper tray floor 15. This step is referred to as the vapour flow period. The duration of the vapour flow period is for example about 30 - 120 seconds.

When the plungers 21 are moved down, the openings 17 in the upper tray floor 15 are opened, while the openings 18 in the lower tray floor 16 are closed off by the lower flanges 23 of the plungers 21. Liquid flows down by gravity from the upper tray floor 15 to the lower tray floor 16, while the vapour flow is stopped. This step is referred to as the liquid-flow period. The liquid flow period takes for example about 1 - 30 seconds. Liquid is collected in the space 25 between the upper and the lower tray floor. When the plungers 21 are moved back upwardly, the liquid flows down to a lower stage 6 (Figure 4C). As a result, liquid and vapour discontinuously flow in cycles step by step.

In the embodiment of Figure 1, the discharged dimethyl ether is fed into the non-cyclic distillation column 3 for further purification and separation of alcohol. Alternatively, the second column 3 can also be a cyclic distillation column. A condenser 26 at the top of the second column 3 discharges dimethyl ether of the desired purity, while a reboiler 27 at the bottom of the second column 3 separates methanol of a high purity.

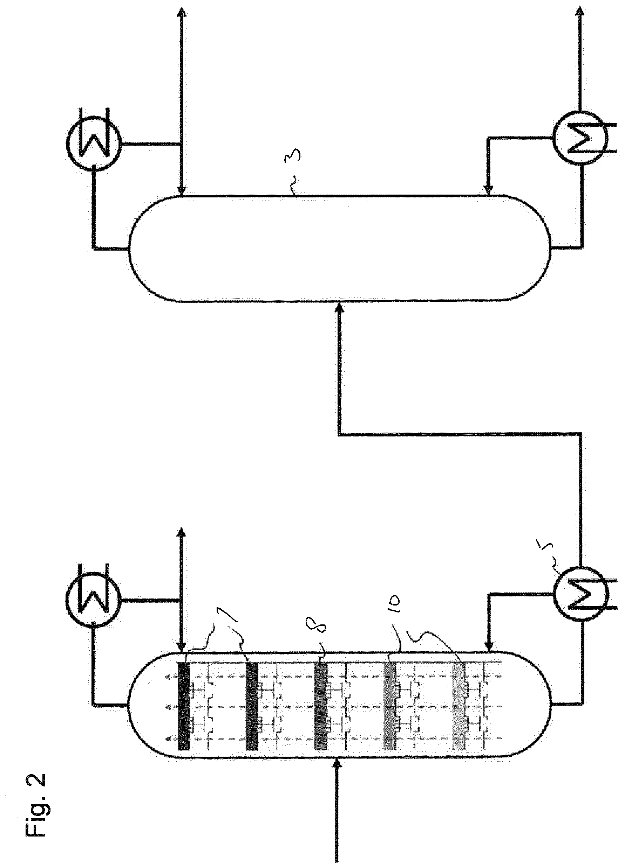

Figure 2 shows an alternative lay-out of an installation for the conversion of methanol to dimethyl ether. The lay-out is similar to the lay-out of Figure 1, but the reflux amount and the vapour flow rate are set such that the temperatures inside the column lead to high-purity dimethyl ether in the top and a methanol-water mixture in the bottoms. The reflux from the condenser is mainly dimethyl ether with trace amounts of methanol. The methanol - water mixture discharged from the reboiler is fed into a non-cyclic distillation column 3 for further purification and separation of water. Alternatively, the second column 3 can also be a cyclic distillation column. A condenser at the top of the second column 3 discharges methanol of the desired purity, while a reboiler at the bottom of the second column 3 separates water of a high purity.

For both installations in Figure 1 and Figure 2 it is possible to recycle the high purity methanol recovered from the column 3 to the feed 11 of the cyclic distillation column 2.

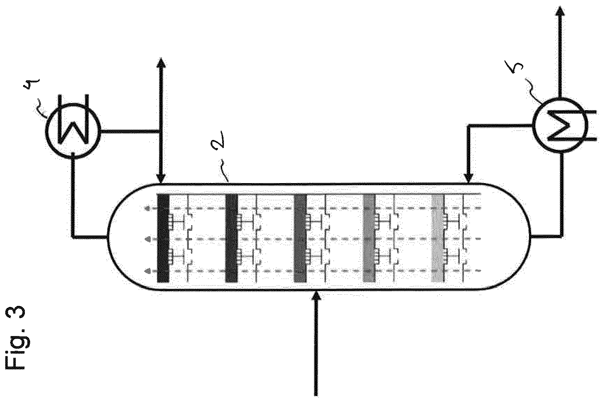

Figure 3 shows a third embodiment of an installation for the production of dimethyl ether by dehydration of methanol. In this embodiment the catalytic cyclic distillation column 2 is configured and operated for full conversion of methanol, resulting in a high-purity dimethyl ether distillate and a high-purity water discharge at the bottom. This can be achieved by adjusting the reflux amount and the vapour flow rate, for example by automatic process control, e.g., using conventional PID controllers. The reflux from the condenser is mainly dimethyl ether with trace amounts of methanol.

### Examples

Performances of the installations of Figures 1, 2, and 3 were calculated using Fortran for the conversion of methanol to dimethyl ether. The reaction kinetics were taken from the reference Hossenininejad S., Afacan A., Hayes R. E., Catalytic and kinetic study of methanol dehydration to dimethyl ether, Chemical Engineering Research and Design, 90 (2012), 825-833, using Amberlyst®-35 as a catalyst. The vapour liquid equilibrium was calculated using activity coefficients derived from the experimental data published in the reference Ye K., Freund H., Sundmacher K., Modelling (vapor+liquid) and (vapor+liquid+liquid) equilibria of {water (H2O) + methanol (MeOH) + dimethyl ether (DME) + carbon dioxide (CO2)} quaternary system using the Peng-Robinson EoS with Wong-Sandler mixing rule, Journal of Chemical Thermodynamics, 43 (2011), 2002-2014.

The input data and the calculated performance parameters are given in Table 1. In Table 1, parameter *"tvap"* stands for the duration of the vapour-flow period, "*xDME*" stands for the dimethyl ether content or purity grade in the distillate, while "*xWater*" stands for the water content in the discharge of the reboiler, referred to as "bottoms".

The design of Figure 2 produced dimethyl ether of a high purity grade of above 99% molar, while the design of Figure 1 produced water of a high purity grade of more than 99.9% molar. The design of Figure 3 produces both dimethyl ether of a high purity grade of above 99% molar and water of a high purity grade of more than 99.9% molar.

**Table 1**

| | Figure 2 (pure DME) | Figure 1 (pure water) | Figure 3 (pure DME and water) |
|---|---|---|---|
| Column Design | | | |
| Rectifying stages | 3 | 3 | 3 |
| Reactive stages | 8 | 8 | 8 |
| Stripping stages | 6 | 6 | 6 |
| Feed stage | 7 | 7 | 7 |
| Catalyst / [kg/stage] | 10 | 10 | 10 |

| Operating conditions | | | |
|---|---|---|---|
| Pressure / [bar] | 10 | 10 | 10 |
| Feed / [kmol/cycle] | 0.15 | 0.15 | 0.15 |
| Vapour flow-rate (reboiler) / [kmol/min] | 1.5 | 1.5 | 1.5 |
| tvap / [min] | 1 | 1 | 1 |
| Reflux amount / [kmol/cycle] | 4.3106 | 4.3106 | 4.8 |
| Temperature in the rectifying section (top stage of the section) | 317.6 K | 396.1 K | 317.6 K |
| Temperature in the stripping section (bottom of section) | 439.6 K | 453.1 K | 453.1 K |

| Column performance | | | |
|---|---|---|---|
| Conversion | 0.895 | 0.943 | 0.996 |
| Distillate / [kmol/cycle] | 0.0675 | 0.079 | 0.0751 |
| xDME in distillate | 0.9945 | 0.8942 | 0.994 |
| Bottoms product / [kmol/cycle] | 0.0825 | 0.071 | 0.0749 |
| XWater in bottoms | 0.8092 | 0.9995 | 0.996 |

## Claims

1. Process for the production of dialkyl ether wherein a C1-C3 alcohol is fed into a distillation column (2) comprising a reboiler (5) at its bottom, a condenser (4) at its top, a feed inlet (11) at a level between the reboiler and the condenser, and a stack of stages (6) in the column's interior, including stages (8) carrying a bed of catalytic material, the stages comprising sluices (19) which are simultaneously moved between a vapour flow position, allowing vapour to flow upwardly through the catalytic beds during a vapour flow period, and a liquid flow position, allowing fluid to flow from an upper stage to a lower stage during a liquid flow period.

2. Process according to claim 1, wherein at least some of the stages (6) comprise a tray with an upper tray floor (15) and a lower tray floor (16) with aligned openings (17), and wherein at least some of the sluices comprise a plunger (21) selectively closing off the opening in the upper tray floor or the opening in the lower tray floor.

3. Process according to claim 1 or 2, wherein the stages (6) include a plurality of rectifying stages (7) near the top of the column, a plurality of stripping stages (10) near the bottom, and a plurality of reactive stages (8) between the rectifying stages on the one hand and the stripping stages on the other hand, the reactive stages being trays carrying a bed of catalytic material.

4. Process according to claim 3, wherein the alcohol is fed into the column (2) at the level of the reactive stages.

5. Process according to any one of the preceding claims, wherein the output of the reboiler (5) and/or the condenser (4) is fed to a second distillation column (3).

6. Process according to any one of the preceding claims using a cyclic distillation column (2) configured for full conversion of the methanol feed.

7. Process according to any one of the preceding claims, wherein the alcohol is or comprises methanol and wherein the dialkyl ether is or comprises dimethyl ether.

8. Installation for the production of a dialkyl ether comprising a distillation column comprising a reboiler (5) at its bottom, a condenser (4) at its top, a feed inlet (11) at a level between the reboiler and the condenser, and a stack of stages (6) in the column's interior, including stages (8) carrying a bed of catalytic material, the stages comprising sluices (19) which are simultaneously moved between a vapour flow position, allowing vapour to flow upwards through the catalytic beds, and a liquid flow position, allowing fluid to flow from an upper stage to a lower stage.

## Patentansprüche

1. Verfahren zur Herstellung von Dialkylether, wobei ein C1-C3 Alkohol in eine Destillierkolonne (2) mit einem Verdampfer (5) an ihrem Boden, einem Kondensator (4) an ihrer Oberseite, einer Beschickungsöffnung (11) auf einer Höhe zwischen dem Verdampfer und dem Kondensator sowie einem Stapel von Stufen (6) im Inneren der Kolonne eingeführt wird, einschließlich Stufen (8), die eine Schicht aus katalytischem Material tragen, wobei die Stufen Schleusen (19) umfassen, welche gleichzeitig zwischen einer Dampfströmungsstellung, die eine Strömung von Dampf nach oben durch die katalytischen Schichten während eines Dampfströmungszeitraumes ermöglicht, und einer Flüssigkeitsströmungsstellung, die eine Strömung von Flüssigkeit von einer oberen Stufe zu einer unteren Stufe während eines Flüssigkeitsströmungszeitraumes ermöglicht, bewegt werden können.

2. Verfahren nach Anspruch 1, wobei zumindest einige der Stufen (6) eine Ablage mit einem oberen Ablageboden (15) und einem unteren Ablageboden (16) mit ausgerichteten Öffnungen (17) umfassen, und wobei zumindest einige der Schleusen einen Kolben (21) umfassen, der wahlweise die Öffnung in dem oberen Ablageboden oder die Öffnung in dem unteren Ablageboden verschließt.

3. Verfahren nach Anspruch 1 oder 2, wobei die Stufen (6) eine Mehrzahl von Korrekturstufen (7) in der Nähe der Oberseite der Kolonne, eine Mehrzahl von Abziehstufen (10) in der Nähe des Bodens sowie eine Mehrzahl von reaktiven Stufen (8) zwischen den Korrekturstufen einerseits und den Abziehstufen andererseits aufweisen, wobei die reaktiven Stufen aus Ablagen bestehen, welche eine Schicht aus katalytischem Material tragen.

4. Verfahren nach Anspruch 3, wobei der Alkohol in die Kolonne (2) auf der Höhe der reaktiven Stufen eingeführt wird.

5. Verfahren nach einem der vorhergehenden Ansprüche, wobei der Ausstoß des Verdampfers (5) und/oder des Kondensators (4) einer zweiten Destillierkolonne (3) zugeführt wird.

6. Verfahren nach einem der vorhergehenden Ansprüche unter Einsatz einer zyklischen Destillierkolonne (2), die zur vollständigen Umwandlung des Methanoleintrags ausgeführt ist.

7. Verfahren nach einem der vorhergehenden Ansprüche, wobei der Alkohol aus Methanol besteht oder Methanol umfasst und wobei der Dialkylether aus Dimethylether besteht oder Dimethylether umfasst.

8. Anlage zur Herstellung von Dialkylether, umfassend eine Destillierkolonne mit einem Verdampfer (5) an ihrem Boden, einem Kondensator (4) an ihrer Oberseite, einer Beschickungsöffnung (11) auf einer Höhe zwischen dem Verdampfer und dem Kondensator sowie einem Stapel von Stufen (6) im Inneren der Kolonne einschließlich Stufen (8), die eine Schicht aus katalytischem Material tragen, wobei die Stufen Schleusen (19) umfassen, welche gleichzeitig zwischen einer Dampfströmungsstellung, die eine Strömung von Dampf nach oben durch die katalytischen Schichten ermöglicht, und einer Flüssigkeitsströmungsstellung bewegt werden, die eine Strömung von Flüssigkeit von einer oberen Stufe zu einer unteren Stufe ermöglicht.

## Revendications

1. Procédé de production d'éther de dialkyle où un alcool en C1 à C3 est introduit dans une colonne de distillation (2) comprenant un rebouilleur (5) au niveau de sa partie inférieure, un condenseur (4) au niveau de sa partie supérieure, une entrée d'alimentation (11) à un niveau entre le rebouilleur et le condenseur, et un empilement d'étages (6) à l'intérieur de la colonne, comportant des étages (8) portant un lit de matériau catalytique, les étages comprenant des vannes (19) qui se déplacent simultanément entre une position d'écoulement de vapeur, permettant à la vapeur de s'écouler vers le haut à travers les lits catalytiques pendant une période d'écoulement de vapeur, et une position d'écoulement de liquide, permettant au fluide de s'écouler d'un étage supérieur à un étage inférieur pendant une période d'écoulement de liquide.

2. Procédé selon la revendication 1, dans lequel au moins certains des étages (6) comprennent un plateau avec un plancher de plateau supérieur (15) et un plancher de plateau inférieur (16) ayant des ouvertures alignées (17), et dans lequel au moins certaines des vannes comprennent un piston plongeur (21) fermant de manière sélective l'ouverture dans le plancher de plateau supérieur ou l'ouverture dans le plancher de plateau inférieur.

3. Procédé selon la revendication 1 ou 2, dans lequel les étages (6) comportent une pluralité d'étages de rectification (7) à proximité de la partie supérieure de la colonne, une pluralité d'étages d'extraction (10) à proximité de la partie inférieure et une pluralité d'étages réactifs (8) entre les étages de rectification d'une part et les étages d'extraction d'autre part, les étages réactifs étant des plateaux portant un lit de matériau catalytique.

4. Procédé selon la revendication 3, dans lequel l'alcool est introduit dans la colonne (2) au niveau des étages réactifs.

5. Procédé selon l'une quelconque des revendications précédentes, dans lequel la sortie du rebouilleur (5) et/ou du condenseur (4) est introduite dans une deuxième colonne de distillation (3).

6. Procédé selon l'une quelconque des revendications précédentes, utilisant une colonne de distillation cyclique (2) configurée pour une conversion totale de la charge de méthanol.

7. Procédé selon l'une quelconque des revendications précédentes, dans lequel l'alcool est ou comprend du méthanol et dans lequel l'éther de dialkyle est ou comprend de l'éther diméthylique.

8. Installation de production d'un éther de dialkyle comprenant une colonne de distillation qui comprend un rebouilleur (5) au niveau de sa partie inférieure, un condenseur (4) au niveau de sa partie supérieure, une entrée d'alimentation (11) à un niveau entre le rebouilleur et le condenseur, et un empilement d'étages (6) à l'intérieur de la colonne, comportant des étages (8) portant un lit de matériau catalytique, les étages comprenant des vannes (19) qui se déplacent simultanément entre une position d'écoulement de vapeur, permettant à la vapeur de s'écouler vers le haut à travers les lits catalytiques, et une position d'écoulement de liquide, permettant au fluide de s'écouler d'un étage supérieur à un étage inférieur.
